# EUROPEAN PATENT SPECIFICATION

(11) **EP 1 598 052 B1**
(45) Date of publication and mention of the grant of the patent: **29.10.2008**
(21) Application number: 04012181.6
(22) Date of filing: 22.05.2004
(51) Int. Cl.: A61K 8/49, A61Q 5/08, A61Q 5/10

(54) **Composition for bleaching and dyeing keratin fibres**
Zusammensetzung zur Aufhellung und Färbung der Keratinfasern
Composition pour éclaircir et teindre les cheveux

(43) Date of publication of application: 23.11.2005
(73) Proprietor: KPSS-Kao Professional Salon Services GmbH, 64297 Darmstadt (DE)
(72) Inventor: Nöcker, Bernd, Dr., 64372 Ober-Ramstadt (DE); Wood, Jonathan, Dr., 69469 Weinheim (DE); Blumenschein, Katrin, 64753 Brombachtal-Heubach (DE)

(56) References cited:
- EP-A- 1 172 082
- WO-A-02/074270
- US-A- 5 188 639

## Description

This invention relates to a two-component bleaching and dyeing composition for keratin fibres, especially hair, comprising in the first part at least one anionic and at least one cationic direct acting hair dyes and in the second part at least one compound with a bleaching and//or brightening effect and showing excellent dyeing and bleaching and/or brightening effects and excellent resistance to hair washing and environmental influences.

Hair colouring and bleaching is a common practice for ages. Oxidative colouration has widely been used for achieving durable, brilliant hair colour. Direct dyes, mainly of cationic character, have also found their applications for colouring hair. Recently, anionic direct dyes have as well been found to be very powerful for changing hair colour permanently and to achieve long lasting, brilliant colours in strong acidic medium. The colouring agents with anionic dyes are so formulated that the optimum conditions are realised for achieving the highest dyestuff penetration into hair. European patent application with laid open number EP 1 022 014 describes such compositions comprising anionic dyestuffs, solvents, as aid to enhance penetration of said dyestuffs, and a buffer solution to adjust the pH of the dyeing agent in the range from 2 to 6. For enhancing penetration of dyestuffs, solvents are used such as ethanol, benzyl alcohol, propylene carbonate, dipropylene glycol. Products are found on the professional hair dressing market applying this technology.

US 5,601,620, as well, discloses hair colouring agents with acid dyes, an organic solvent and at least one polysiloxane as a conditioner. The dyeing compositions disclosed here are having a pH in the range of 1.5 - 4.5.

Above two documents deal with the anionic dyes in acidic medium and they are silent on any colouring and/or lightening compositions at an alkaline pH and optionally comprising an oxidizing agent.

In an earlier European Patent application from our company with the application number 04 001 799.8, hair colouring composition is disclosed based on only anionic acidic direct dyes at alkaline medium. In that application, the possibility of addition of cationic dyestuff is disclosed to be only at minor quantities, preferably is not advised.

EP 810 851, on contrary to the above two publications, discloses hair colouring and lightening compositions comprising a cationic direct dye and an oxidizing agent. This documents is totally silent on any composition comprising anionic dyes.

EP 920 856 discloses a process for bleaching and colouring hair in two steps. The document is silent on using any anionic direct dyes in the compositions.

WO 02/074270 A1 discloses compositions for simultaneously lightening and colouring hair with cationic and anionic dyes. Some anionic dyes such as Acid red 52 are disregarded in said application because of poor stability in the presence of a bleach.

US 5 188 639 discloses compositions for simultaneously dyeing and waving hair with fluorescein dyes.

EP 1 172 082 A2 discloses hair compositions with cationic dyes of the azo-pyridinium type.

In practice, further development is obviously desired by professional hair dressing practitioners and also by end consumers for achieving highly brilliant and long lasting colorations and bleaching and brightening preferably in a single process. Additionally, colour resistance against washing and environmental influences is highly desirable. Furthermore, partial colourations in form of streaks has become popular and products are desired showing optimal colouring and at the same time optimal lightening performance for colourful hair appearance.

This invention starts with the above mentioned problems and discloses a hair colouring and bleaching composition with excellent colouring and bleaching effects together with excellent stability against washing (shampooing) and environmental influences. The compositions of the present invention are also excellently suitable for achieving partial bleaching and colouring in the form of streaks in a single process.

The composition of the present invention is a two part agent which are to be mixed homogeneously prior to application onto hair, preferably as well with an agent comprising at least one oxidizing agent, comprising a water-free composition being part A which comprises at least one compound with bleaching and/or brightening effect and an aqueous composition being Part B comprising at least one direct acting anionic dyestuff and at least one direct acting cationic dyestuff as more explicitly defined in part B under. The compositions of the present invention show excellent colouring and bleaching effects and the achieved colorations are excellently stable against washing (shampooing) and environmental influences.

### Part A - water-free powder composition

According to the present invention, in the part A being the water free composition, suitable compounds with bleaching and/or brightening effects are in general peroxides Useful as such are in particular persulfates such as sodium and potassium persulfate, ammonium persulfate, earth alkali peroxides such as magnesium peroxide, melamine peroxide or urea peroxide or phtholimidoperoxy- hexanoic acid. The proportion of peroxides is at least 10 %, preferably at least 20 % to about 80 %, calculated to the total powder composition - only Part A.

According to the invention, the Part A, water free composition, of the compositions can also comprise 0.1 % to 10 % by weight, calculated to the total powder composition - Part A, of one or more ammonium salts.

Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium artrate, ammonium benzoate, ammonium acetate, ammonium formiate and ammonium lactate.

Preferred thereof are the ammonium phosphates, such as NH₄H₂PO₄, (NH₄)₂HPO₄, (NH₄)₂NaPO₄, NaNH₄HPO₄ or NH₄Na₂PO₄, ammonium chloride, ammonium sulfate and diammonium hydrogen citrate, as well as ammonium chloride, preferably in an amount from 0.1 % to 10 % by weight, calculated to the total powder composition - Part A.

As known from EP 609 796 A2, the ammonium compounds can also be used as sole bleaching agent in respectively higher amounts.

The proportion of the bleaching or brightening compounds in total preferably ranges from about 5 % to about 75 %, in particular about 25 % and about 60 % by weight, in reference to the brightening and bleaching powder - only Part A.

### Part B - Aqueous composition

According to the invention the suitable direct acting anionic dyes in Part B of the composition are:

Acid Red 52, DC Violet 2, DC Red 33, DC Orange 4, DC Red 27 and DC Yellow 10.

The cationic dyestuffs with the following chemical structures are the ones according to the present invention. 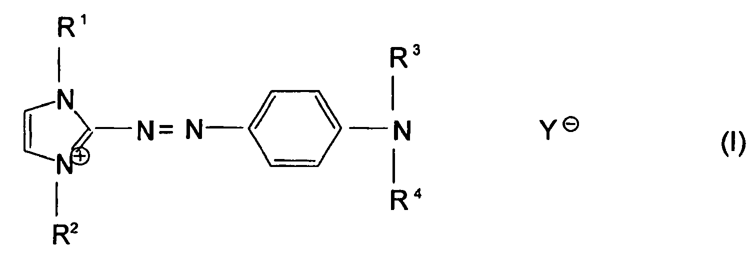 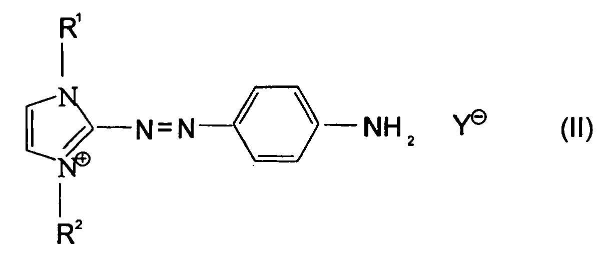 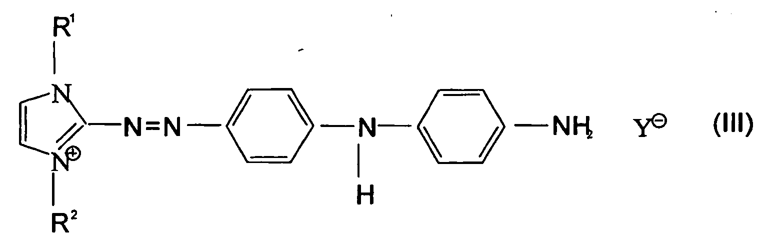 and 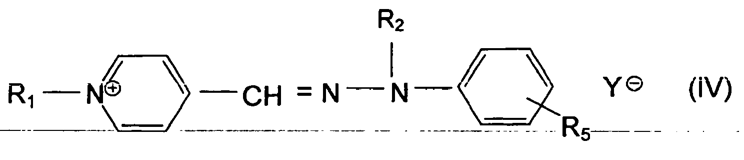 wherein R¹, R², R³ and R⁴ stand for hydrogen, a CH₃- or C₂H₅- group, R⁵ stands for hydrogen, -OCH₃ or -OC₂H₅ and Y is an anion such as chloride, bromide, methosulfate.

The most preferred compounds are the ones according to the formula I, where R₁. R₂, R₃ and R₄ are methyl and Y is chloride, according to formula II where R₁ and R₂ are methyl and Y is chloride and according to the formula IV, where R₁ and R₂, are methyl, R₅ is hydrogen and Y is methosulfate.

Additionally other cationic dyestuffs can as well be used in addition to the cationic dyestuffs mentioned above. Some examples to those are:

Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

Suitable cationic dyestuffs are in principal those available on the market for cosmetic hair colouring applications. For this purpose, special reference is made to the PCT application WO 95/15144 of Ciba-Geigy AG.

According to the invention, bleaching and coloring composition - Part B - comprises anionic dyes at a concentration of 0.1 to 7.5%, preferably 0.2 to 5%, more preferably 0.2 to 3% by weight calculated to total aqueous composition - Part B. Cationic dyestuffs are included into the compositions of the present invention at a concentration of 0.01 to 7.5%, preferably 0.05 to 5% and more preferably 0.05 to 3% by weight calculated to total aqueous composition - Part B.

Interestingly it has as well been found out that the ratio of acidic dyes to cationic dyes plays an important role for achieving especially resistance to washing and environmental effects. Accordingly, the ratio of cationic dyestuffs to acidic dyestuffs by weight is in the range of 3:1 to 1:10, preferably 2: 1 to 1:7 and further more preferably 2:1 to 1:5.

Additionally, the coloring compositions of the present invention may comprise neutral dyes (HC dyes), so called nitro dyes for shading purposes. Concentration of those can typically be in the range from 0.01 to 2.5%, preferably 0.1 to 2% by weight calculated to total aqueous composition - Part B.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

pH of the aqueous composition of the present invention - Part B - varies between 5 and 12, preferably 6-11, more preferably 6.8 to 10. pH of the aqueous composition - Part B - is adjusted to the required pH by using triethanolamine, ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

Aqueous composition - Part B - of present invention can comprise additionally in the base formulation fatty acids with 0 to 3 ethylenic bonds and with fatty acyl chain length of 12 to 22 C atom. Concentration of the fatty acids can be in the range of 0.1 to 10%, preferably 0.1 to 7.5% and most preferably 0.2 to 5% by weight calculated to the total composition. Fatty acid examples, without limiting the choice, suitable for colouring compositions are myristic acid, palmitic acid, behenic acid, steraic acid, oleic acid, linoleic acid. The most preferred fatty acid is oleic acid.

Aqueous compositions - Part B - according to the present invention can be in the form of emulsion, solution, dispersion and/or gel. Emulsion form is preferred.

In the case that the colouring composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 20%, preferably 0.5 to 15% by weight, calculated to total composition.

Colouring compositions according to present invention comprises surfactants selected from anionic, nonionic, amphoteric (or zwiterionic) and/or cationic surfactants as emulsifier or solubilizer. Cationic surfactants are as well used as hair conditioners in the colouring composition.

Anionic surfactants suitable within the scope of the invention are in principal known from the cleansing compositions and are preferably present in an amount from 0.1 to about 10%, preferably 0.2 to 7.5% and most preferably 0.2 - 5% by weight, calculated to the total composition.

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₆- (C₂H₄O)ₙ- O - CH₂COOX,

wherein R₆ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 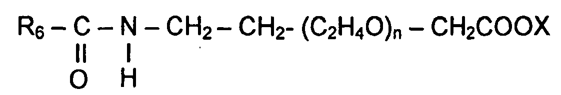 wherein R₆ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants in a mixture.

An overview of the anionic surfactants suitable for the present invention can furthermore be found in the monography of K. Schrader, "Grundlagen und Rezepturen der Kosmetika", 2nd Ed.(1989, Hüthig Buchverlag), pp. 595-600 and pp. 683 to 691.

Further surfactants in the colouring compositions according to the invention are nonionic surfactants alone or in admixture with anionic surfactants at a weight ratio of 3:1 to 1:3.

These are described as well in Schrader, I.c., on pages 600-601 and pp. 694-695.

Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide.

Further nonionic surfactants suited are alkyl polyglucosides of the general formula

R₇-O-(R₃O)ₙ-Zₓ,

wherein R₇ is an alkyl group with 8 to 18 carbon atoms, R₃ is an ethylene or propylene group, Z is a saccharide group with 5 to 6 carbon atoms, n is a number from 0 to 10 and x is a number between 1 and 5.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

As further surfactant component, the colouring compositions according to the invention can also contain amphoteric or zwitterionic surfactants, for example in an amount from about 0.5 % to about 5 %, preferably from about 1 % to about 2.5 % by weight, calculated to the total composition.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structures 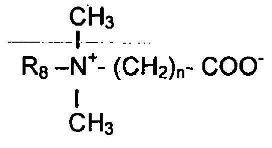 and 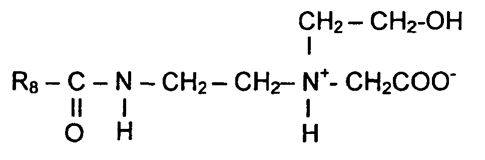 wherein R₈ is a C₈-C₁₈-alkyl group and n is 1 to 3;
sulfobetaines of the structure 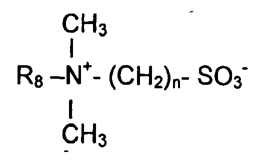 wherein R₈ and n are same as above;
and amidoalkyl betaines of the structure 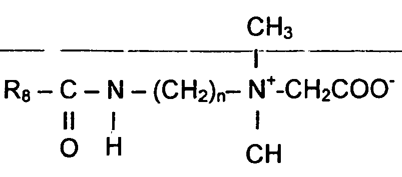 wherein R₈ and n are same as above.

Colouring composition can contain cationic surfactans as emulsifier, solubilizer and/or conditioning ingredients according to the formula, but not limited to. 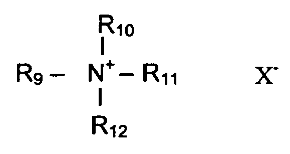 where R₉ is a saturated or unsaturated, branched or non-branched alkyl chain with 8-22 C atoms or

R₁₃ CO NH (CH₂)ₙ

where R₁₃ is saturated or unsaturated, branched or non-branched alkyl chain with 7-21 C atoms and n has typical value of 1 - 4 or

R₁₄ CO O (CH₂)ₙ

where R₁₄ is saturated or unsaturated, branched or non-branched alkyl chain with 7-21 C atoms and n has typical value of 1 - 4, and
R₁₀ is H or unsaturated or saturated, branched or non-branched alkyl chain with 1-22 C atoms or

R₁₃ CO NH (CH₂)ₙ

or

R₁₄ CO O (CH₂)ₙ

where R₁₃, R₁₄ and n are same as above.

R₁₁ and R₁₂ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The following compounds can be added either into the water free Part-A or to the aqueous Part B of the compositions of the present invention. The concentrations given are valid for only Part A and/or Part B.

Colouring composition can also contain cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Typical concentration range for any of the cationic conditioners mentioned above can be 0.01 - 5% by weight, preferably 0.03 - 2.5% by weight and more preferably 0.05 - 1.5% by weight.

Hair dyeing and bleaching composition of the present invention preferably comprise an organopolysiloxane wherein at least one silicium atom is linked to an alkylene group having a hetero-atom, in particular a nitrogen atom, with a poly-(N-acyl alkyleneimine) units of the formula 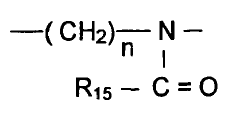 wherein n is a number from 1 to 5 and R₁₅ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 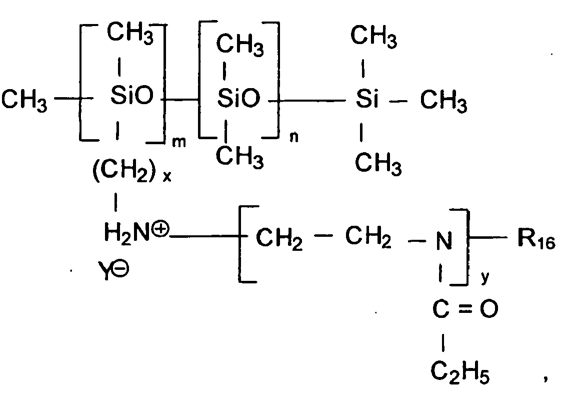 wherein m and n each are numbers from 20 to 10,000, in particular 50 to 7,000, especially 100 to 5,000, x is a number between 1 and 5, preferably 3, and y is a number from 5 to 30, R₁₆ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Colouring compositions according to the present invention can contain organic solvents as penetration enhancers and also as a solubilzers. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 10%, preferably 0.5 - 5% by weight calculated to the total composition. Obviously those are preferably contained in the aqueous composition Part B.

The hair dyeing compositions according to the invention preferably contain thickening agents. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Optionally, the composition of this invention can comprise further hair conditioning agents such as silicone oils either volatile or non-volatile, natural and synthetic oils. Among silicone oils those can be added to the colouring composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Additional non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula X or XI, respectively,

R₁₇ CO (O CH₂ CH₂)ₙ OH

R₁₇ CO (O CH₂ CH₂)ₙ O OC R₁₈

where R₁₇ and R₁₈ are independent from each other saturated, unsaturated or branched or non-branched alkyl chain with 7 to 21 C atoms and n is typically 2 - 100.

Another preferred compound in the composition is of ceramide type of compounds according to general formula 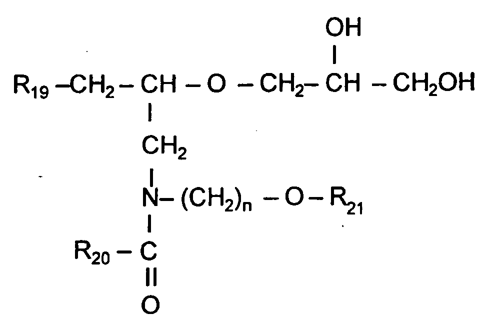 where R₁₉ and R₂₀ are independent from each other alkyl- or. alkenyl group mit 10 to 22 carbon atoms, R₂₁ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1% by weight calculated to the total composition.

The compositions of the present invention either in Part A or Part B can comprise of at least one ubiquinone of the formula (I) 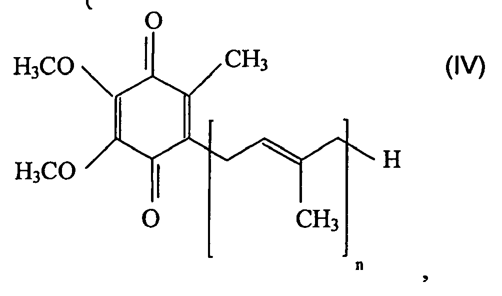 wherein n is a number from 1 to 10. Concentration of ubichinone can vary between 0.001 % and 10 % by weight, calculated to the total of the part A or B of the composition.

Colouring composition may as well contain UV filters of oil soluble, non-ionic, ones and/or as well those of water soluble and mainly of anionic character. Examples are Benzophenone-1 Benzophenone-2, Benzophenone-3, Benzophenone-7, Benzophenone-6, Berizophenone-8, octylmethoxy cinnamate, homosalat to those of oil soluble ones and Benzophenone-4, benzophenone-9 to those anionic water soluble ones. It should be noted that the other UV filters of oil and water soluble ones should as well be possible to combine.

The mixing ratio of the Part A and B, depending on the colour result to be achieved varies between 5:1 to 1:5, preferably 3:1 to 1:5 and more preferably 2:1 to 1:3, all by weight.

Another preferred way of carrying out the present invention is that mixing compositions with an agent, Part C, comprising at least one oxidizing agent prior to application onto hair. The oxidizing agents suitable are hydrogen peroxide, urea peroxide, melamin peroxide or perborate salts. The most preferred is hydrogen peroxide, which is used as a lotion containing 3 to 12% by weight, calculated to composition only comprising hydrogen peroxide.

The new composition as a result of mixing Parts A and B and oxidizing composition Part C allows achieving simultaneous intensive bleaching and coloring. The mixing ratio of the composition resulted from the combination of the two parts of the present invention (Part AB) and oxidizing composition (Part C) should be in the range of 4:1 to 1:4, by weight, preferably 3:1 to 1:3 by weight, more preferably 2:1 to 1:3 by weight..

### Process for colouring and bleaching

Among many different possibilities of application, one way of achieving bleaching and colouring effect that the parts A and B are mixed in a ratio given above and applied onto hair and left on the hair for 5 to 45 min and rinsed of from hair and shampooed if necessary. During the processing time on the hair heat can as well be applied which should not exceed 45°C. Heat application may result in shortening of the processing time.

Another way of carrying out hair bleaching and colouring in one step is that the Parts A and B are combined in a ratio give above and further into the composition Part C - oxidizing agent comprising composition - is added again at a ratio disclosed above and the resulting mixture is applied onto hair and left on the hair for 5 to 45 min and rinsed of from hair and shampooed if necessary. During the processing time on the hair heat can as well be applied which should not exceed 45°C. Heat application may result in shortening of the processing time.

Although the preferred process for colouring and bleaching is a single step process, two-step processes may as well be used with the compositions of the present invention. One of the ways of carrying out in two steps is that in the first step, composition according to Part A of the present invention is firstly mixed with an oxidizing agent - Part C - and applied onto hair and left 5 to 20 min and afterwards without rinsing off the dyestuff comprising Part B is applied onto hair and additionally processed for 5 to 30 min and rinsed off from hair and shampooed, if necessary. During the processing time in both steps on the hair heat can as well be applied which should not exceed 45°C. Heat application may result in shortening of the processing time.

In another way of carrying out the invention in two step is that in the first step, composition according to Part A of the present invention is firstly mixed with an oxidizing agent - Part C - and applied onto hair and left 5 to 45 min and afterwards rinsed off from hair and subsequently in the second step the Part B comprising dyeing agents is applied onto hair and additionally processed for 5 to 45 min and rinsed off from hair and shampooed, if necessary. During the processing time in both steps on the hair heat can as well be applied which should not exceed 45°C. Heat application may result in shortening of the processing time.

Still another way of carrying out the invention is that in the first step Part B comprising the dyestuffs is applied onto hair and left 5 to 30 min on the hair and without rinsing it off from the hair, applying the composition resulting in mixing of Part A with an oxidizing agent Part C onto hair and processing additionally 5 to 45 min and rinsing off from hair and shampooing, if necessary. During the processing time in both steps on the hair heat can as well be applied which should not exceed 45°C. Heat application may result in shortening of the processing time.

### Optionals

Another way of carrying out the invention is that addition of oxidation dyestuffs precursors (developing substances) and coupling substances into Part B of the composition of the present invention. Those oxidative dyes can as well be mixed into the colouring composition prior to application onto hair. It is possible to incorporate developing substances known **per se.** In the case of oxidation dyes are present in the compositions, colouring is than carried out in the presence of oxidizing agent, i.e. oxidative dye containing colouring composition is mixed with oxidizing agent prior to application. Special mention is made of p-phenlynediamine, p-methylaminophenol and substituted p-phenylenediamines such as 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxypyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

The total concentration of the developing substances customarily ranges between about 0.05 % and 5 %, preferably 0.1 % and 4 %, in particular 0.1 % to 3 % by weight, calculated to the total hair dyeing composition (excluding the oxidation agent), whereby these figures are always related to the proportion of free base.

The composition according to the present invention can contain coupling substances, which can be selected from 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl resorcinol, 4-chiororesorcinoi, 2-amino-4-chiorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, a-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition (excluding the oxidizing agent), whereby these figures are always related to the proportion of free base.

The composition of the present invention can contain additional ingredients such as preservatives, chelating agents, fragrance and substances customarily used in cosmetic bleaching and colouring compositions of keratin fibres, especially hair.

The invention is illustrated with the following examples, but not limited to.

### Example 1

### Bleaching and coloring composition

| **Part A** | |
|---|---|
| Hydroxyethylcellulose | 1.40% by weight |
| Cellulose gum | 3.10 |
| Xanthan gum | 0.30 |
| Tetrasodium EDTA | 3.00 |
| Sodium carbonate | 0.90 |
| Ammonium persulfate | 17.00 |
| Potassium persulfate | 35.00 |
| Sodium metasilicate | 8.00 |
| Corn starch | 7.20 |
| Diatomaceous Earth | 13.00 |
| Polyquaternium - 10 | 0.10 |
| Mineral oil | 9.20 |

The composition is prepared in the same way as disclosed in EP 560 088 A1.

| **Part B** | |
|---|---|
| | % by weight |
| | I |
| Cocamide MEA | 4.00 |
| Cetearyl alcohol | 10.00 |
| Tegin P | 1.40 |
| Propylene Glycol | 2.40 |
| Oleic acid | 3.00 |
| Coenzyme Q10 | 0.10 |
| Ammonium chloride | 0.50 |
| Tetrasodium EDTA | 0.20 |
| Sodium lauryl sulfate | 1.50 |
| Organopolysiloxane A1 of EP 640 643 | 0.20 |
| Ceramide according to formula wherein | |
| R₁₈and R₁₉ are C16 and R₂₀ is ethyl | 0.10 |
| DC Red 27 | 0.50 |
| Compound according to formula I R1=R2=R3=R4=methyl, Y=chloride | 0.50 |
| | |
| Water | to 100 |

The above composition Part B is as well prepared only with DC Red 27 and the dyestuff according to formula I with R1=R2=R3=R4=methyl, Y=chloride for comparative purposes.

The part A and B are mixed 1:1, by weight, and mixed as well with 1 part of 6% hydrogen peroxide lotion with the following composition.

| **Part C- Hydrogen Peroxide Lotion** | |
|---|---|
| Hydrogen peroxide | 6.00 (% by wt.) |
| Cetyl stearyl alcohol | 1.70 |
| Phosphoric acid | 0.50 |
| Sodium lauryl sulfate | 0.20 |
| Coenzyme Q10 | 0.05 |
| Cetyl PG hydroxyethyl palmitamide | 0.10 |
| Disodium hydrogen phosphate | 0.10 |
| Salicylic acid | 0.10 |
| Water | ad 100.00 |

The homogeneous mixture so obtained was applied onto hair and left 30 min at 40°C and rinsed off with water and shampooed.

**Table I: ΔE values after bleaching and coloring of hair**

| | ΔE |
|---|---|
| Inventive composition | 55 |
| | |
| Comparative composition (only with DC Red 27) | 51 |
| | |
| Comparative composition (only with compound of formula I R1=R2=R3=R4=methyl, Y=chloride) | 47 |

In order show color fastness against washing, an intensive red colored hair tress with a composition comprising DC red 52 and cationic red dye according to formula I with R₁=R₂=R₃=R₄= methyl, as presented above, was washed 10 times under usual hair wash conditions with a commercial shampoo composition designed for colored hair of the trade mark Goldwell Definition, and color of the tresses (L, a and b values) were measured before and after shampooing optically with a laboratory equipment and color differences were calculated with the well known equation to obtain ΔE values and color intensity differences (ΔL) were obtained from measured L values. The results are presented in Table II below. The test was as well carried out with compositions comprising only the cationic dyestuff of formula I with R₁=R₂=R₃=R₄= methyl, at a concentration as incorporated into the inventive coloring composition and at a concentration equal to the total dyestuff content of the inventive composition (sum of concentrations anionic and cationic dyestuffs).

**Table II: Results of durability test against wash - shampooing**

| | ΔE | ΔL |
|---|---|---|
| Inventive composition 0.5% cationic and 0.5% anionic dyestuffs | 6.1 | 2.8 |
| | | |
| Comparative with 0.5% cationic dye | 14.5 | 7.4 |
| | | |
| Comparative with 1 % cationic dye | 12.4 | 6.6 |

The results in the table II should be understood as the lower the value, the better the colourfastness against washing. Thus, the most stable color is obtained with the inventive composition.

Dyestuff composition examples which are added into the Part B.

**Table III**

| | Intensive red | % by weight Violet-red |
|---|---|---|
| Acid Red 52 | 1.50 | - |
| DC Violet 2 | - | - |
| DC Red 33 | - | 1.50 |
| DC Orange 4 | - | - |
| Dyestuff of formula I R₁=R₂=R₃=R₄= methyl | 0.50 | 0.50 |

**Table IV**

| | % by weight Orange (reddish) | Intensive red |
|---|---|---|
| Acid Red 52 | - | 0.60 |
| DC Violet 2 | - | |
| DC Red 33 | - | - |
| DC Orange 4 | 1.50 | - |
| Dyestuff of formula I R₁=R₂=R₃=R₄= methyl | 0.50 | 1.20 |

**Table V**

| | Orange | % by weight orange-red | Violet- red |
|---|---|---|---|
| Acid Red 52 | - | 1.50 | - |
| DC Violet 2 | - | - | - |
| DC Red 33 | - | - | 1.5 |
| DC Orange 4 | 1.50 | - | - |
| Dyestuff of formula II R₁=R₂= methyl | 0.50 | 0.50 | 0.50 |

**Table VI**

| | Yellow | % by weight Shiny orange red | Orange |
|---|---|---|---|
| Acid Red 52 | - | 1.50 | - |
| DC Yellow10 | 1.50 | - | - |
| DC Red 33 | - | - | - |
| DC Orange 4 | - | - | 1.50 |
| Dyestuff of formula IV | 0.50 | 0.50 | 0.50 |
| R₁=R₂= methyl and R₃=H and Y is methosulfate | | | |

**Table VII**

| | % by weight Orange (reddish) |
|---|---|
| Acid Red 52 | - |
| DC Yellow 10 | - |
| DC Red 33 | 1.50 |
| DC Orange 4 | - |
| Dyestuff of formula IV | 0.50 |
| R₁=R₂ =methyl and R₃=H and Y is methosulfate | |

### Example 2

### Bleaching and coloring composition

| **Part A** | |
|---|---|
| Silicon dioxide | 16.17 (% by wt.) |
| Ammonium chloride | 14.70 |
| Sodium carbonate | 10.00 |
| Sodium metasilicate | 2.30 |
| Phthalimidoperoxyhexanic acid | 41.50 |
| Sodium persulfate | 15.20 |
| Coenzyme Q10 | 0.10 |
| Cetyl PG hydroxyethyl palmitamide | 0.10 |
| DC Red 27 | 1.50 |
| Compound according to formula I R1=R2=R3=R4=methyl, Y=chloride | 0.50 |

| **Part B** | |
|---|---|
| Cetyl stearyl alcohol | 11.0 (% by wt.) |
| Oleth-5 | 5.0 |
| Oleic acid | 2.5 |
| Stearamide MEA | 2.3 |
| Cocoamide MEA | 2.3 |
| Sodium cetyl stearyl sulfate | 1.2 |
| 1.2-Propyleneglycol | 1.0 |
| 1.2-Propyleneglycol stearate | 0.6 |
| Sodium lauryl sulfate | 0.5 |
| Wheat protein hydrolyzate | 0.9 |
| Organopolysiloxane | 0.4 |
| Cetyl PG hydroxyethyl palmitamide | 0.1 |
| Panthenol | 0.6 |
| Perfume | 0.4 |
| Complexing agent | 0.2 |
| DC Red 27 | 1.5 |
| Compound according to formula I R1=R2=R3=R4=methyl, Y=chloride | 0.5 |
| Water | ad 100.0 |

This composition is mixed in a weight proportion of Part A:B:C 1:2.5:2.5. The Part C is the same formulation as the one in Example 1. The pH-value of this mixture was 9.0. Upon application this mixture showed an excellent, even coloring effect with good wet and dry combability and excellent gloss, and improved elasticity; substantial hair damage was not apparent on strands of undamaged human hair.

The same example was as well used only by mixing Part A and B at a weight ratio of 1:1 and applied onto hair. Excellent coloring performance was observed in terms of uniformity and brilliance.

## Claims

1. Composition for keratin fibers, especially for hair **characterized in that** it is a two-part composition to be mixed homogeneously prior to application comprising a water free composition being Part A which comprises at least one compound with bleaching effect and an aqueous composition being part B which comprises at least one acidic direct dye selected from Acid Red 52, DC Violet 2, DC Red 33, DC Orange 4, DC Red 27 and DC Yellow 10 and at least one cationic direct dye selected from compounds presented with general formula 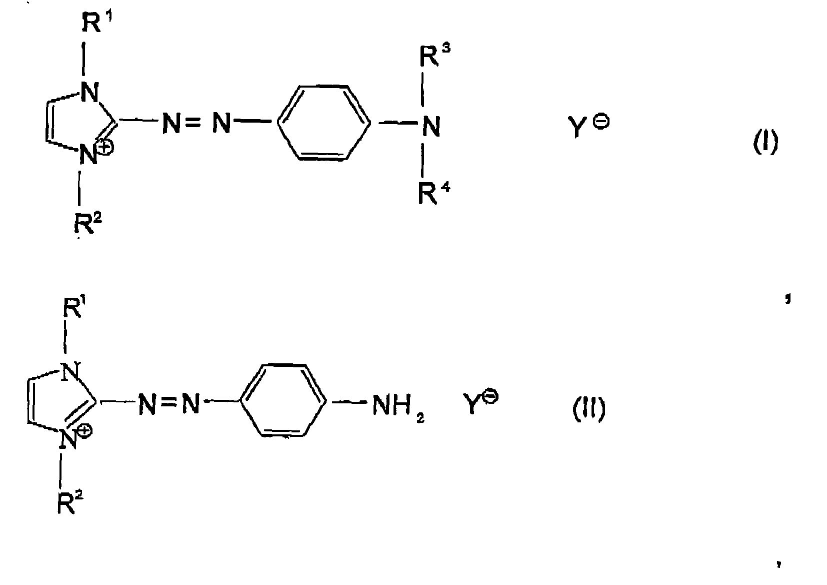 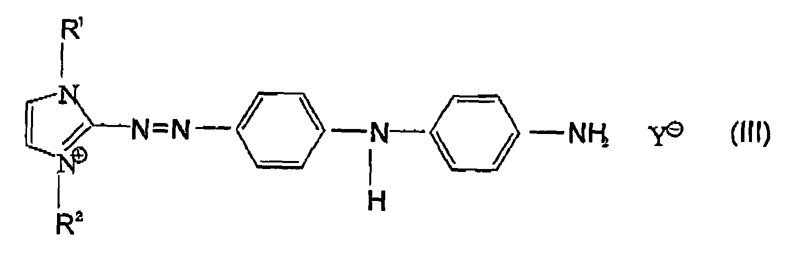 and 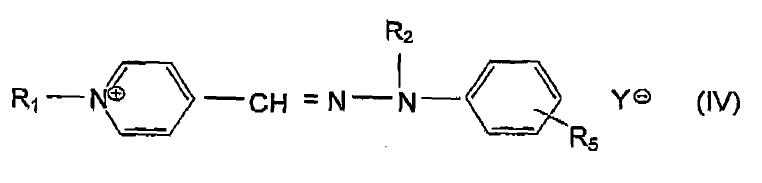 wherein R¹, R², R³ and R⁴ stand for hydrogen, a CH₃- or C₂H₅- group, R⁵ stands for hydrogen, -OCH₃ or -OC₂H₅ and Y is an anion such as chloride, bromide, methosulfate,

2. Composition according to claim 1 **characterized in that** it comprises a third part being Part C comprising at least one oxidizing agent.

3. Composition according to claims 1 and 2 **characterized in that** cationic dyestuff is 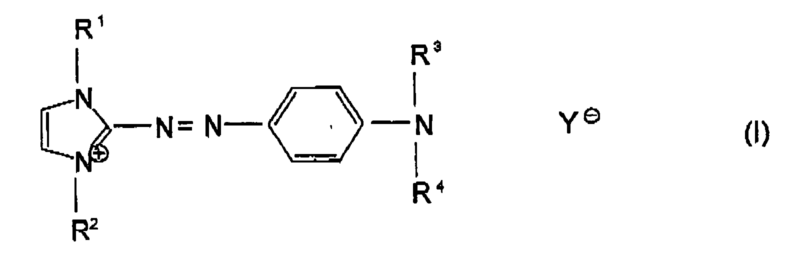 wherein R¹, R², R³ and R⁴ are CH₃- group, and Y is chloride.

4. Composition according to claims 1 and 2 **characterized in that** cationic dyestuff is 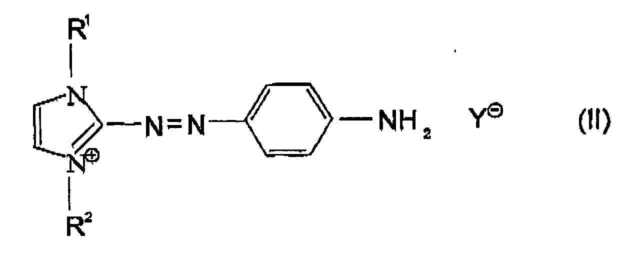 wherein R¹ and R² re CH₃- group, and Y is chloride.

5. Composition according to claims 1 and 2 **characterized in that** cationic dyestuff is 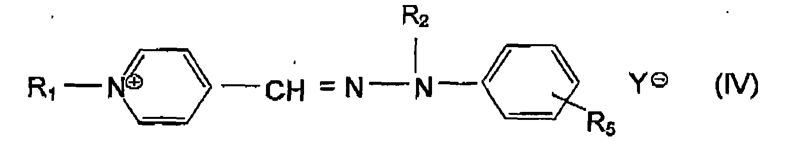 wherein R¹ and R^{2 a}re CH₃- group, R₅ is hydrogen and Y is methosulfate.

6. Composition according to any of the preceding claims **characterized in that** pH of the composition Part B is between 5 and 12.

7. Composition according to any of the preceding claims **characterized in that** pH of the composition Part B is between 6.8 and 10.

8. Composition according to any of the preceding claims **characterized in that** it comprises at least one cationic dyestuff and at least one acidic dyestuff In Part B at a weight ratio of 3:1 to 1:10, preferably 2:1 to 1:7.

9. Composition according to any of the preceding claims **characterized in that** it comprises anionic direct dye in Part B at a concentration of 0.1 - 7.5% by weight, calculated to the total composition.

10. Composition according to any of the preceding claims **characterized in that** it comprises cationic direct dye in Part B at a concentration of 0.01 -7.5% by weight, calculated to the total composition.

11. Composition according to any of the preceding claims **characterized in that** it comprises in Part B HC dyes in addition to anionic and cationic direct dyes.

12. Composition according to any of the preceding claims **characterized in that** it comprises in Part B an additional cationic direct dye.

13. Composition according to any of the preceding claims **characterized in that** it comprises in Part A at least one persulfate and/or peroxo compounds as bleaching compound

14. Composition according to any of the preceding claims **characterized in that** it comprises in Part A at least one ammonium salt as a compound with bleaching effect.

15. Composition according to any of the preceding claims **characterized in that** it comprises organosiloxane polymer in either Part A or Part B according to formula 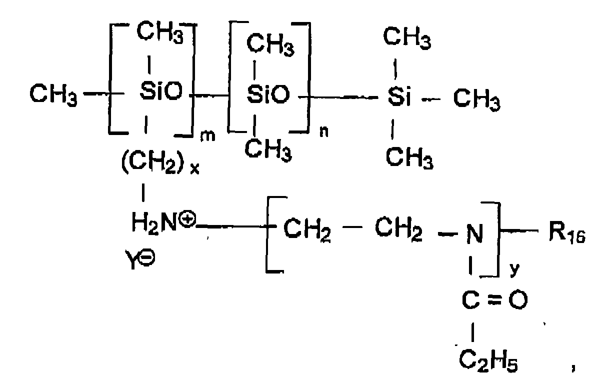 wherein m and n each are numbers from 20 to 10,000, x is a number between 1 and 5, and y is a number from 5 to 30, R₁₆ is a C₁-C₁₂-alkyl or aryl group, in particular a methyl, ethyl or benzyl group, and Y⁻ is an anion.

16. Composition according to any of the preceding claims **characterized in that** it comprises additionally ceramide type compound in either Part A or Part B according to the formula 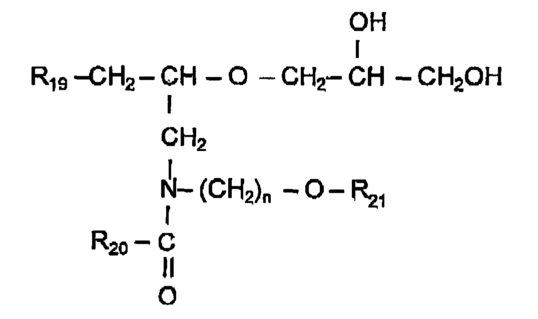 where R₁₉ and R₂₀ are independent from each other alkyl- or alkenyl group mit 10 to 22 carbon atoms, R₂₁ is methyl, ethyl, n-propyl or isopropyl group and n is a number between 1 to 6, preferably 2 or 3.

17. Composition according claim 2 **characterized in that** oxidizing agent is hydrogen peroxide at a concentration of 3 to 12% by weight calculated to total composition.

18. Composition according any of the preceding claims **characterized in that** it comprises additionally oxidative dyes precursors and/or coupling substances.

19. Use of composition according to any of the preceding claims for colouring and bleaching of keratin fibres, especially hair.

20. Process for colouring and bleaching keratin fibres, especially hair **characterised in that** the parts A and B according to claims 1 to 1-18 are mixed homogeneously at a ratio of 5:1 to 1:5, by weight (Part A : Part B) and applied onto hair and left on the hair for 5 to 45 min with or without heat application where the temperature not exceeding 45°C and rinsed of from hair.

21. Process for colouring and bleaching keratin fibres, especially hair **characterised in that** the parts A and B according to claims 1 to 18 are mixed homogeneously at a ratio of 5:1 to 1:5, by weight (Part A: Part B) with another agent comprising at least one oxidizing agent Part C and applied onto hair and left on the hair for 5 to 45 min with or without heat application where the temperature not exceeding 45°C and rinsed of from hair.

22. Process for colouring and bleaching keratin fibres, especially hair, **characterised in that** the parts A according to claims 1 to 18 is mixed homogeneously with an oxidizing agent comprising composition (Part C) and applied onto hair and left on the hair for 5 to 30 min with or without heat application where the temperature not exceeding 45°C and without rinsing off from hair Part B is applied and left on the hair for additional 5 to 30 min with or without heat application where the temperature not exceeding 45°C and rinsed off,

23. Process for colouring and bleaching keratin fibres, especially hair, **characterised in that** the parts A according to claims 1 to 18 is mixed homogeneously with an oxidizing agent comprising composition (Part C) and applied onto hair and left on the hair for 5 to 45 min with or without heat application where the temperature not exceeding 45°C and rinsed off from hair, and subsequently Part B is applied and left on the hair for additional 5 to 45 min with or without heat application where the temperature not exceeding 45°C and rinsed off.

24. Process for colouring and bleaching keratin fibres, especially hair, **characterised in that** the parts B according 9 to claims 1 to 18 is applied onto hair and left on the hair for 5 to 30 min with or without heat application where the temperature not exceeding 45°C and without rinsing it off from hair, a mixture of Part A according to claims 1 to 18 and an agent comprising at least one oxidizing agent mixed homogeneously (Part C) and applied onto hair and left on the hair for 5 to 45 min with or without heat application where the temperature not exceeding 45°C and rinsed off from hair, and subsequently rinsed off.

## Patentansprüche

1. Zusammensetzung für Keratin-Fasern, besonders für Haare, **dadurch gekennzeichnet, dass** sie eine 2-Komponenten- Zusammensetzung ist, die vor der Applikation homogen vermischt wird, enthaltend eine wasserfreie Zusammensetzung als Teil A, welche mindestens eine Verbindung mit einem Bleicheffekt enthält, und eine wässrige Zusammensetzung als Teil B, welche mindestens einen sauren direkt ziehenden Farbstoff, ausgewählt aus Acid Red 52, DC Violet 2, DC Red 33, DC Orange 4, DC Red 27 und DC Yellow 10, und mindestens einen kationischen direkt ziehenden Farbstoff ausgewählt aus Verbindungen, dargestellt mit der allgemeinen Formel 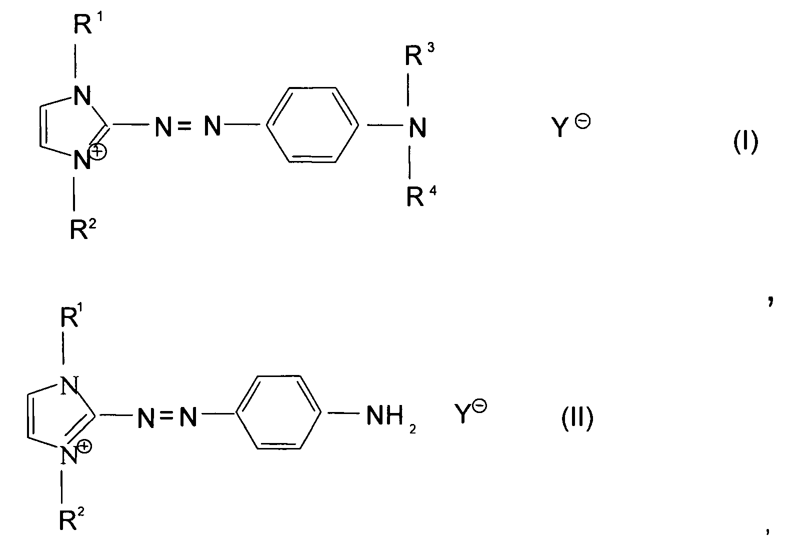 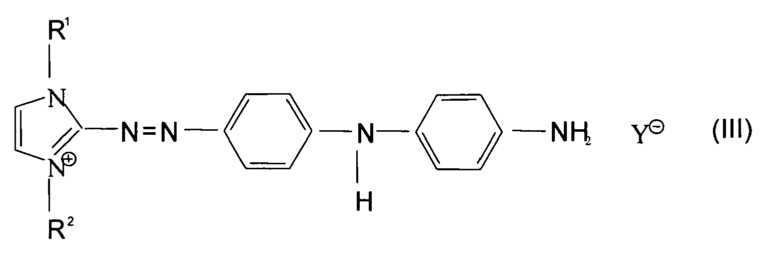 und 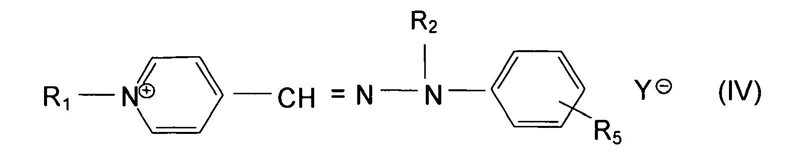 worin R¹, R², R³ und R⁴ für Wasserstoff, eine CH₃- oder C₂H₅- Gruppe stehen, R⁵ für Wasserstoff, -OCH₃ oder -OC₂H₅ steht, und Y ein Anion wie Chlorid, Bromid und Methosulfat ist, enthält.

2. Zusammensetzung nach Anspruch 1, **dadurch gekennzeichnet, dass** sie einen dritten Teil als Teil C enthält, welcher mindestens ein Oxydationsmittel enthält.

3. Zusammensetzung nach den Ansprüchen 1 und 2, **dadurch gekennzeichnet, dass** der kationische Farbstoff 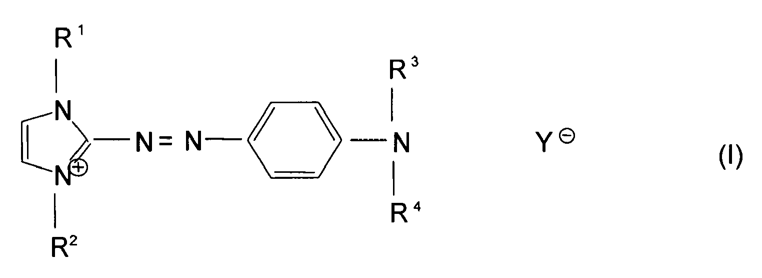 ist,
worin R¹, R², R³ und R⁴ CH₃- Gruppen sind, und Y Chlorid ist.

4. Zusammensetzung nach den Ansprüchen 1 und 2, **dadurch gekennzeichnet, dass** der kationische Farbstoff 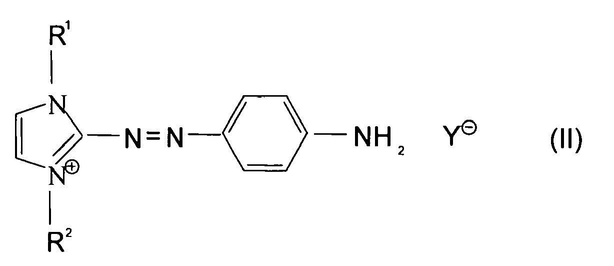 ist,
worin R¹ und R² CH₃- Gruppen sind, und Y Chlorid ist.

5. Zusammensetzung nach den Ansprüchen 1 und 2, **dadurch gekennzeichnet, dass** der kationische Farbstoff 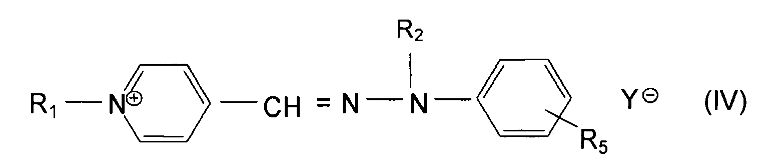 ist,
worin R¹ und R² CH₃- Gruppen sind, R₅ Wasserstoff ist, und Y Methosulfat ist.

6. Zusammensetzung nach einem der vorhergehenden Ansprüche, **dadurch gekennzeichnet, dass** der pH-Wert vom Teil B der Zusammensetzung zwischen 5 und 12 liegt.

7. Zusammensetzung nach einem der vorhergehenden Ansprüche, **dadurch gekennzeichnet, dass** der pH-Wert vom Teil B der Zusammensetzung zwischen 6,8 und 10 liegt.

8. Zusammensetzung nach einem der vorhergehenden Ansprüche, **dadurch gekennzeichnet, dass** sie in Teil B mindestens einen kationischen Farbstoff und mindestens einen sauren Farbstoff in einem Gewichtsverhältnis von 3:1 bis 1:10, vorzugsweise 2:1 bis 1:7, enthält

9. Zusammensetzung nach einem der vorhergehenden Ansprüche, **dadurch gekennzeichnet, dass** sie anionische, direkt ziehende Farbe in Teil B in einer Menge von 0,1 - 7,5% Gew.- %, berechnet auf die Gesamtzusammensetzung, enthält.

10. Zusammensetzung nach einem der vorhergehenden Ansprüche, **dadurch gekennzeichnet, dass** sie kationische, direkt ziehende Farbe in Teil B in einer Menge von 0,01 - 7,5% Gew.- %, berechnet auf die Gesamtzusammensetzung, enthält.

11. Zusammensetzung nach einem der vorhergehenden Ansprüche, **dadurch gekennzeichnet, dass** sie in Teil B, zusätzlich zu anionischen und kationischen Farben, HC Farben enthält.

12. Zusammensetzung nach einem der vorhergehenden Ansprüche, **dadurch gekennzeichnet, dass** sie in Teil B zusätzlich eine kationische direkt ziehende Farbe enthält.

13. Zusammensetzung nach einem der vorhergehenden Ansprüche, **dadurch gekennzeichnet, dass** sie in Teil A mindestens eine Persulfat- und/oder Peroxo- Verbindungen als Bleichmittel enthält.

14. Zusammensetzung nach einem der vorhergehenden Ansprüche, **dadurch gekennzeichnet, dass** sie in Teil A mindestens ein Ammoniumsalz als Verbindung mit einem Bleicheffekt, enthält.

15. Zusammensetzung nach einem der vorhergehenden Ansprüche, **dadurch gekennzeichnet, dass** sie Organosiloxanepolymer in einem der Teile A oder B enthält, gemäß der Formel 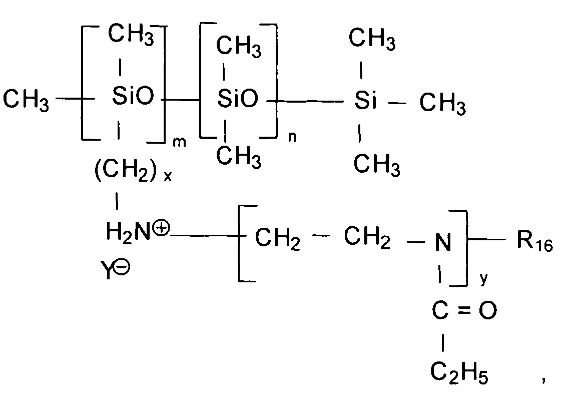 worin m und n jeweils Zahlen von 20 to 10,000 sind, x eine Zahl zwischen 1 and 5 ist, und y eine Zahl von 5 bis 30 ist, R₁₆ eine C₁-C₁₂-Alkyl- oder Aryl- Gruppe, insbesondere eine Methyl-, Ethyl- oder Benzyl- Gruppe ist, und Y- ein Anion ist.

16. Zusammensetzung nach einem der vorhergehenden Ansprüche, **dadurch gekennzeichnet, dass** sie zusätzlich eine Ceramid-Verbindung in einem der Teile A oder B enthält, gemäß der Formel 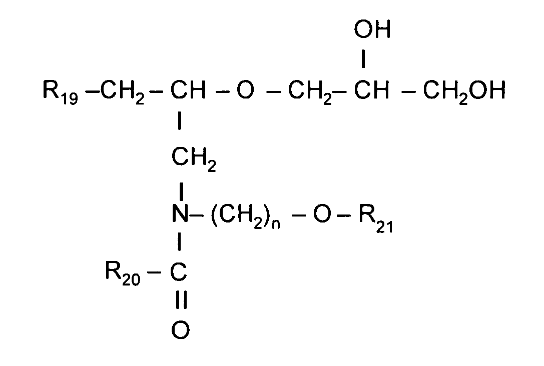 wobei R₁₉ und R₂₀ unabhängig voneinander Alkyl- oder AlkenylGruppen mit 10 bis 22 Kohlenstoffatomen sind, R₂₁ eine Methyl-, Ethyl- , n-Propyl- oder Isopropyl-Gruppe ist, und n eine Zahl zwischen 1 bis 6, vorzugsweise 2 oder 3 ist.

17. Zusammensetzung nach Anspruch 2, **dadurch gekennzeichnet, dass** das Oxydationsmittel Wasserstoffperoxyd ist, in einer Konzentration von 3 bis 12 Gew.- %, berechnet auf die Gesamtzusammensetzung.

18. Zusammensetzung nach einem der vorhergehenden Ansprüche, **dadurch gekennzeichnet, dass** sie zusätzlich Entwickler- und/oder Kuppler-Sustanzen von Oxydationsfarbstoffen, enthält.

19. Verwendung der Zusammensetzung nach einem der vorhergehenden Ansprüche zum Färben und Bleichen von Keratinfasern, besonders von Haaren.

20. Verfahren zum Färben und Bleichen von Keratinfasern, besonders von Haaren, **dadurch gekennzeichnet, dass** die Teile A und B nach den Ansprüchen 1 bis 18 in einem Gewichtsverhältnis von 5:1 bis 1:5 (Teil A : Teil B) homogen gemischt werden, auf das Haar aufgetragen werden, und für 5 bis 45 Minuten mit oder ohne Wärmezufuhr, wobei die Temperatur nicht über 45°C steigen soll, auf dem Haar bleiben, und vom Haar abgespült werden.

21. Verfahren zum Färben und Bleichen von Keratinfasern, besonders von Haaren, **dadurch gekennzeichnet, dass** die Teile A und B nach den Ansprüchen 1 bis 18 in einem Gewichtsverhältnis von 5:1 bis 1:5 (Teil A : Teil B) mit einem anderen Mittel, Teil C, enthaltend mindestens ein Oxydationsmittel, homogen gemischt werden, auf das Haar aufgetragen werden, und für 5 bis 45 Minuten mit oder ohne Wärmezufuhr, wobei die Temperatur nicht über 45°C steigen soll, auf dem Haar bleiben, und vom Haar abgespült werden.

22. Verfahren zum Färben und Bleichen von Keratinfasern, besonders von Haaren, **dadurch gekennzeichnet, dass** die Teile A nach den Ansprüchen 1 bis 18 homogen mit einer Oxydationsmittel enthaltenden Zusammensetzung (Teil C) homogen gemischt werden, und auf das Haar aufgetragen werden, und für 5 bis 30 Minuten mit oder ohne Wärmezufuhr, wobei die Temperatur nicht über 45°C steigen soll auf dem Haar bleiben, und ohne vom Haar abgespült zu werden, der Teil B aufgetragen wird und für weitere 5 bis 30 Minuten mit oder ohne Wärmezufuhr, wobei die Temperatur 45°C nicht übersteigen soll, auf dem Haar bleibt, und dann vom Haar abgespült wird.

23. Verfahren zum Färben und Bleichen von Keratinfasern, besonders von Haaren, **dadurch gekennzeichnet, dass** die Teile A nach den Ansprüchen 1 bis 18 homogen mit einer Oxydationsmittel enthaltenden Zusammensetzung (Teil C) homogen gemischt werden, und auf das Haar aufgetragen werden und für 5 bis 45 Minuten mit oder ohne Wärmezufuhr, wobei die Temperatur nicht über 45°C steigen soll auf dem Haar bleiben, und dann vom Haar abgespült werden, anschließend der Teil B aufgetragen wird und für zusätzliche 5 bis 45 Minuten mit oder ohne Wärmezufuhr, wobei die Temperatur 45°C nicht übersteigen soll, auf dem Haar bleibt, und dann vom Haar abgespült wird.

24. Verfahren zum Bleichen und Färben von Keratinfasern, besonders von Haaren, **dadurch gekennzeichnet, dass** die Teile B nach den Ansprüchen 1 bis 18 auf die Haare aufgetragen werden und für 5 bis 30 Minuten, mit oder ohne Wärmezufuhr, wobei die Temperatur 45°C nicht übersteigen soll, auf dem Haar bleiben, und ohne vom Haar abgespült zu werden wird eine homogene Mischung aus Teil A, nach den Ansprüchen 1 bis 18, und einem Mittel, enthaltend mindestens ein Oxydationsmittel (Teil C), auf das Haar aufgetragen und für 5 bis 45 Minuten mit oder ohne Wärmezufuhr, wobei die Temperatur 45°C nicht übersteigen soll auf dem Haar belassen, und anschließend abgespült.

## Revendications

1. Composition pour des fibres de kératine, en particulier pour des cheveux **caractérisée en ce qu'**il s'agit d'une composition en deux parties à mélanger de manière homogène avant l'application, comprenant une composition sans eau qui est la partie A laquelle comprend au moins un composé avec un effet blanchissant et une composition aqueuse qui est la partie B laquelle comprend au moins un colorant direct acide choisi parmi l'Acid Red 52, le DC Violet 2, le DC Red 33, le DC Orange 4, le DC Red 27 et le DC Yellow 10 et au moins un colorant direct cationique choisi parmi les composés présentés avec la formule générale 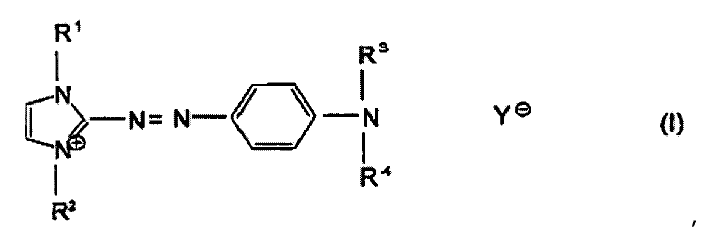 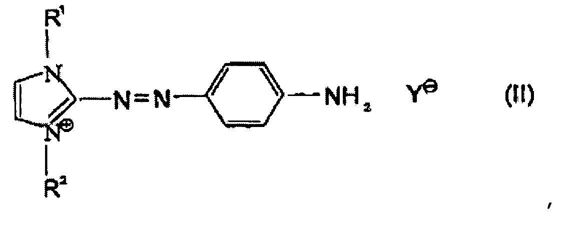 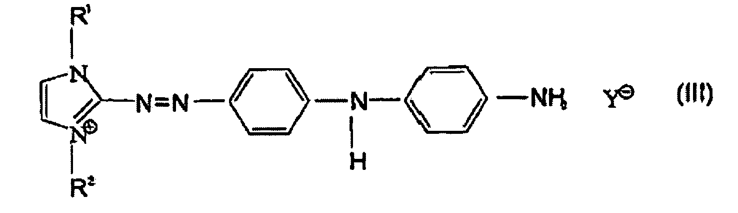 et 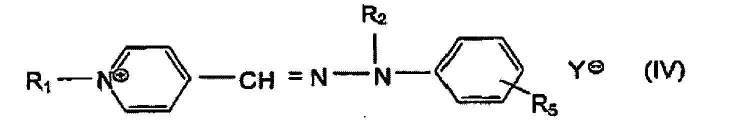 dans lesquelles R¹, R², R³ et R⁴ représentent un atome d'hydrogène, un groupe CH₃- ou C₂H₅-, R⁵ représente un atome d'hydrogène, un groupe -OCH₃ ou -OC₂H₅ et Y représente un anion tel qu'un chlorure, un bromure, un méthosulfate.

2. Composition selon la revendication 1, **caractérisée en ce qu'**elle comprend une troisième partie qui est la partie C comprenant au moins un agent oxydant.

3. Composition selon les revendications 1 et 2, **caractérisée en ce que** la matière colorante cationique est 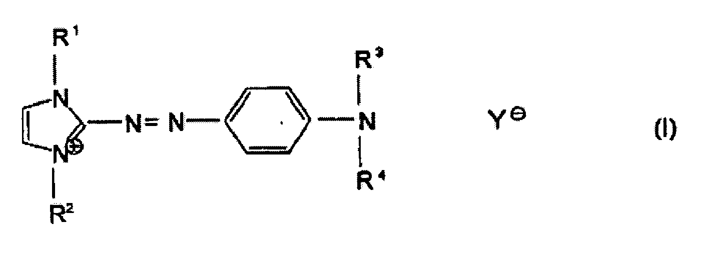 dans laquelle R¹, R², R³ et R⁴ représentent un groupe CH₃-, et Y représente un chlorure.

4. Composition selon les revendications 1 et 2, **caractérisée en ce que** la matière colorante cationique est 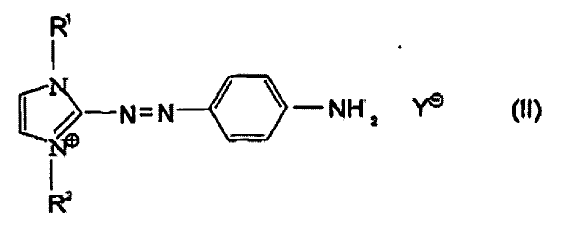 dans laquelle R¹ et R² représentent un groupe CH₃-, et Y représente un chlorure.

5. Composition selon les revendications 1 et 2, **caractérisée en ce que** la matière colorante cationique est 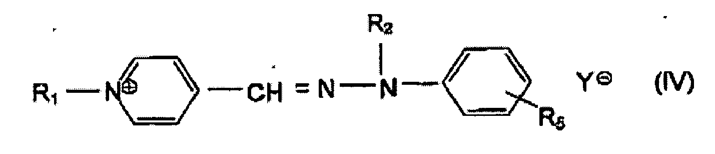 dans laquelle R¹ et R² représentent un groupe CH₃-, R₅ représente un atome d'hydrogène et Y représente un méthosulfate.

6. Composition selon l'une quelconque des revendications précédentes, **caractérisée en ce que** le pH de la partie B de la composition est entre 5 et 12.

7. Composition selon l'une quelconque des revendications précédentes, **caractérisée en ce que** le pH de la partie B de la composition est entre 6,8 et 10.

8. Composition selon l'une quelconque des revendications précédentes, **caractérisée en ce qu'**elle comprend au moins une matière colorante cationique et au moins une matière colorante acide dans la partie B dans un rapport pondéral de 3:1 à 1:10, de préférence de 2:1 à 1:7.

9. Composition selon l'une quelconque des revendications précédentes, **caractérisée en ce qu'**elle comprend un colorant direct anionique dans la partie B à une concentration de 0,1 à 7,5 % en poids, calculé par rapport à la composition totale.

10. Composition selon l'une quelconque des revendications précédentes, **caractérisée en ce qu'**elle comprend un colorant direct cationique dans la partie B à une concentration de 0,01 à 7,5 % en poids, calculé par rapport à la composition totale.

11. Composition selon l'une quelconque des revendications précédentes, **caractérisée en ce qu'**elle comprend dans la partie B des colorants HC en plus des colorants directs anioniques et cationiques.

12. Composition selon l'une quelconque des revendications précédentes, **caractérisée en ce qu'**elle comprend dans la partie B, un colorant direct cationique supplémentaire.

13. Composition selon l'une quelconque des revendications précédentes, **caractérisée en ce qu'**elle comprend dans la partie A au moins un composé persulfate et/ou peroxo en tant que composé décolorant.

14. Composition selon l'une quelconque des revendications précédentes, **caractérisée en ce qu'**elle comprend dans la partie A au moins un sel d'ammonium en tant que composé avec un effet décolorant.

15. Composition selon l'une quelconque des revendications précédentes, **caractérisée en ce qu'**elle comprend un polymère organosiloxane soit dans la partie A soit dans la partie B selon la formule 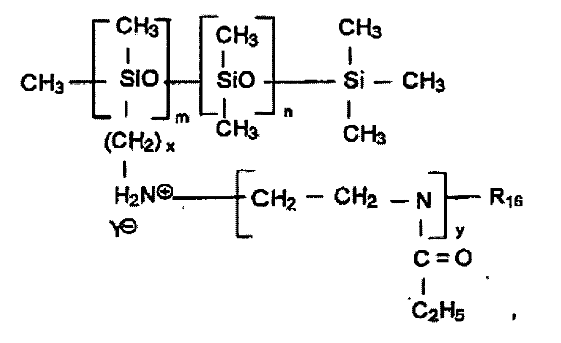 dans laquelle m et n sont chacun des nombres de 20 à 10 000, x est un nombre entre 1 et 5, et y est un nombre de 5 à 30, R₁₆ est un groupe alkyle en C₁-C₁₂ ou aryle, en particulier un groupe méthyle, éthyle ou benzyle, et Y⁻ est un anion.

16. Composition selon l'une quelconque des revendications précédentes, **caractérisée en ce qu'**elle comprend en plus un composé de type céramide soit dans la partie A soit dans la partie B selon la formule 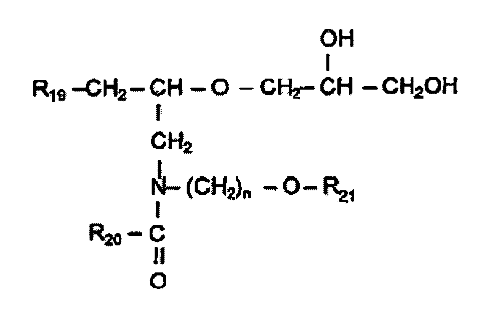 où R₁₉ et R₂₀ représentent de manière indépendante l'un de l'autre un groupe alkyle- ou alcényle de 10 à 22 atomes de carbone, R₂₁ représente un groupe méthyle, éthyle, n-propyle ou isopropyle et n est un nombre entre 1 et 6, de préférence 2 ou 3.

17. Composition selon la revendication 2, **caractérisée en ce que** l'agent oxydant est du peroxyde d'hydrogène à une concentration de 3 à 12 % en poids calculée par rapport à la composition totale.

18. Composition selon l'une quelconque des revendications précédentes, **caractérisée en ce qu'**elle comprend en plus des précurseurs de colorants par oxydation et/ou des substances de couplage.

19. Utilisation d'une composition selon l'une quelconque des revendications précédentes pour la coloration et la décoloration de fibres de kératine, en particulier de cheveux.

20. Procédé pour colorer et décolorer des fibres de kératine, en particulier des cheveux, **caractérisé en ce que** les parties A et B selon les revendications 1 à 18 sont mélangées de manière homogène dans un rapport de 5:1 à 1:5, en poids (partie A:partie B) et appliquées sur les cheveux et laissées sur les cheveux pendant 5 à 45 minutes avec ou sans application de chaleur où la température ne dépasse pas 45°C et rincées des cheveux.

21. Procédé pour colorer et décolorer des fibres de kératine, en particulier des cheveux **caractérisé en ce que** les parties A et B selon les revendications 1 à 18 sont mélangées de manière homogène dans un rapport de 5:1 à 1:5, en poids (partie A:partie B) avec un autre agent comprenant au moins une partie C d'agent oxydant et appliquées sur les cheveux et laissées sur les cheveux pendant 5 à 45 minutes avec ou sans application de chaleur où la température ne dépasse pas 45°C et rincées des cheveux.

22. Procédé pour colorer et décolorer des fibres de kératine, en particulier des cheveux **caractérisé en ce que** la partie A selon les revendications 1 à 18 est mélangée de manière homogène avec une composition comprenant un agent oxydant (partie C) et appliquée sur les cheveux et laissée sur les cheveux pendant 5 à 30 minutes avec ou sans application de chaleur où la température ne dépasse pas 45°C et sans rinçage des cheveux, la partie B est appliquée et laissée sur les cheveux pendant 5 à 30 minutes supplémentaires avec ou sans application de chaleur où la température ne dépasse pas 45°C et rincée.

23. Procédé pour colorer et décolorer des fibres de kératine, en particulier des cheveux **caractérisé en ce que** la partie A selon les revendications 1 à 18 est mélangée de manière homogène avec une composition comprenant un agent oxydant (partie C) et appliquée sur les cheveux et laissée sur les cheveux pendant 5 à 45 minutes avec ou sans application de chaleur où la température ne dépasse pas 45°C et rincée des cheveux, et par la suite la partie B est appliquée et laissée sur les cheveux pendant 5 à 45 minutes supplémentaires avec ou sans application de chaleur où la température ne dépasse pas 45°C et rincée.

24. Procédé pour colorer et décolorer des fibres de kératine, en particulier des cheveux, **caractérisé en ce que** la partie B selon les revendications 1 à 18 est appliquée sur les cheveux et laissée sur les cheveux pendant 5 à 30 minutes avec ou sans application de chaleur où la température ne dépasse pas 45°C et sans la rincer des cheveux, un mélange de partie A selon les revendications 1 à 18 et d'un agent comprenant au moins un agent oxydant mélangé de manière homogène (partie C) et appliqué sur les cheveux et laissé sur les cheveux pendant 5 à 45 minutes avec ou sans application de chaleur où la température ne dépasse pas 45°C et rincé des cheveux, et par la suite rincé.
